# EUROPEAN PATENT APPLICATION

(11) **EP 1 985 293 A1**
(43) Date of publication of application: **29.10.2008**
(21) Application number: 07707764.2
(22) Date of filing: 31.01.2007
(51) Int. Cl.: A61K 31/19, A61K 35/20, A61P 35/00, A61P 35/02, A61P 43/00, C07K 1/02, C07K 14/76

(54) **METHOD FOR PRODUCING DENATURED SUBSTANCE OF LACTALBUMIN**

(30) Priority: 01.02.2006 JP 2006024102
(71) Applicant: National University Corporation Hokkaido University, Sapporo-shi, Hokkaido 060-0808 (JP)
(72) Inventor: DEMURA, Makoto, Kita-ku, Sapporo-shi, Hokkaido 060-0810 (JP); AKIMOTO, Kaoru, Kita-ku, Sapporo-shi, Hokkaido 060-0810 (JP); AIZAWA, Tomoyasu, Kita-ku, Sapporo-shi, Hokkaido 060-0810 (JP); KAWANO, Keiichi, Kita-ku, Sapporo-shi, Hokkaido 060-0810 (JP)
(74) Representative: Hiebl, Inge Elisabeth
(86) International application number: PCT/JP2007/051546
(87) International publication number: WO 2007/088869

(57) **Abstract**

It is intended to provide a method for producing a denatured substance of lactalbumin, particularly the substance showing a cell death inducing activity against a tumor cell, simply at a low cost. The method for producing a denatured substance of lactalbumin includes heating a mixture of domestic animal milk and oleic acid. The method of the invention can produce a useful substance as a pharmaceutical which induces cell death of a tumor cell such as solid cancer or a leukemic lymphocyte from domestic animal milk which is an extremely inexpensive raw material and has advantages that the raw material can be obtained easily, supply of the raw material is stable, the raw material is inexpensive and the like. Further, the method is industrially advantageous because it has characteristics that a large amount of the tumor cell death inducing substance can be produced by a batch reaction, a column treatment is not required and the like.

## Description

### Technical Field

The present invention relates to a method for the inexpensive and efficient production of a substance from the milk of domestic animals which exhibits cell death inducing activity against tumor cells.

### Background Art

It is known that among the proteins contained in the milk of mammals, there are proteins which obtain, when subjected to specific treatment, an activity of suppressing proliferation of harmful cells as represented by tumor cells by inducing cell death against the cells. For example, α-lactalbumin oligomerized in the molten globule-like state, which can induce cell death (apoptosis) in tumor cells, has been obtained by converting human lactalbumin to be in the molten globule-like state, and applying the resultant to an ion exchange column onto which a fatty acid or casein fraction is immobilized (Patent Document 1 and Non-Patent Document 1).

However, the production of a tumor cell death inducing substance in the prior art requires a method necessitating complicated processes such as multiple pre-treatments including mixing with a calcium chelating agent or the like, or the immobilization of lipids or the like onto ion exchange columns. In the prior method, the lactalbumin to be used should be recombinantly produced because the lactalbumin is a human lactalbumin and the prior method has some further deficiencies such as that the ion exchange column used in the production of the tumor cell death inducing substance is expensive, that the production efficiency of the substance is low, and that column treatment which is an operation difficult to be achieved in mass production is required. Thus, the prior method needs extensive improvements in order to be applied to industrial production of the tumor cell death inducing substance and the method is not therefore considered to be practical.
Patent Document 1: Japanese Patent Application National Publication No. 2001-524491
Non-Patent Document 1: Svensson et al., Protein Science, Vol. 12, pp. 2794-2804 (2003)

### Disclosure of the Invention

### Problems to be Solved by the Invention

It is an object of the present invention to develop a new method capable of mass producing the denatured products of lactalbumin, particularly a tumor cell death inducing substance which induces cell death in tumor cells, the method including a selection of more easily available and inexpensive raw materials.

### Means for Solving the Problems

The inventors of the present invention paid attention to using the milk of domestic animals, particularly cow's milk, which is more easily available and inexpensive, as the raw materials, and found that the denatured products of lactalbumin, particularly a tumor cell death inducing substance which induces apoptosis in tumor cells, can be produced by a batch type method without using a column or the like. Thus, the inventors completed the following inventions.

(1) A method for producing the denatured products of lactalbumin, the method including heating a mixture of milk of a domestic animal and oleic acid.

(2) Themethodaccordingto (1), wherein the milk is skimmed milk.

(3) The method according to (1), wherein the domestic animal is a cow.

(4) The method according to (1), wherein the mixture is heated at a temperature of 50°C to 60°C.

(5) The method according to (1), further including subjecting the mixture obtained after the heating to centrifugation to recover an aqueous layer containing a tumor cell death inducing substance.

(6) The method according to any one of (1) to (5), wherein the method produces the denatured products of lactalbumin exhibiting cell death inducing activity against tumor cells.

### Effects of the Invention

The method of the present invention is capable of producing a substance which is useful as a medicine for inducing cell death in tumor cells such as solid tumors and leukemic lymphocytes, from a very cheap raw materials, that is, the milk of domestic animals, and has advantages such as easy availability, stable supply and low price of the raw materials. The method is also a method which is industrially advantageous in that a tumor cell death inducing substance can be produced in large amounts by a batch type reaction, a column treatment is not required, and the like.

### Best Mode for Carrying Out the Invention

The denatured products of lactalbumin as used in the present invention means denatured lactalbumin which prepared according to the production method of the present invention. As shown in the Examples described later, a simple mixture of lactalbumin and oleic acid does not exhibit the activity of inducing cell death in tumor cells as exhibited by the denatured products of lactalbumin prepared by the method of the present invention. Therefore, it is suspected that the denatured products of lactalbumin in the present invention is a lactalbumin, when heated in the presence of oleic acid, which is in a state different from naturally occurring lactalbumin, particularly in a state of being conjugated to oleic acid via covalent bonding through a reaction between the functional groups of the lactalbumin and oleic acid, or being conj ugated via non-covalent bonding other than hydrophobic bonding, or being conjugated in some other form.

Furthermore, the tumor cell death inducing substance as used in the present invention refers to a substance which can impede or interrupt the proliferative potential of tumor cells, or can kill the cells, when the substance is brought in a condition for allowance of contact with tumor cells laid in a proliferative environment.

The function of the tumor cell death inducing substance (activity of inducing cell death in tumor cells) as used in the present invention may be measured by selecting appropriate tumor cells, for example, L1210 cells, as the object cells; adding the tumor cell death inducing substance produced by the method of the present invention to a system in which these tumor cells are proliferated in an appropriate culture medium and conditions, and then measuring the survival rate or cell proliferation rate of the tumor cells.

With regard to the milk of a domestic animal that can be used in the present invention, any milk can be used as long as it is the milk of a mammal grown for a purpose of producing milk, such as cow, sheep or goat. However, from the viewpoint of the supply amount of the raw materials or that a stable massive supply system has been established, cow's milk is most preferred.

According to the present invention, the denatured products of lactalbumin, particularly a cell death inducing substance which induces cell death in tumor cells, can be produced very conveniently by heating a mixture of the milk of a mammal and oleic acid.

The milk used in the present invention does not particularly require various pre-treatments which are required in the method described in Patent Document 1, for example, operations such as adding a chelating agent, making the pH acidic, or preliminary heating. On the other hand, it has been confirmed that when skimmed milk is used as the raw materials, even if the amount of addition of oleic acid is reduced, the final amount of the cell death inducing substance does not change. Therefore, the use of the skimmed milk as the raw materials increases the production efficiency for the denatured products of lactalbumin, particularly the cell death inducing substance which induces cell death in tumor cells, with respect to the amount of oleic acid used. The milk skimming operation may be performed by a general method such as a centrifugation treatment, and is not particularly limited.

Heating of the milk added with oleic acid may be performed at 40°C to 60°C, and preferably at 50°C to 60°C. Although the treatment temperature is further increased, no significant change has been recognized in the activity of the denatured products of lactalbumin of the present invention in inducing cell death in tumor cells. The heating time depends on the amount of milk used, but generally about 10 minutes to 2 hours may be favorable. The amount of addition of oleic acidmay be adjusted in accordance with the nature of the milk used, but preferably 40 µl or more of oleic acid may be added relative to 1 ml of whole milk, while generally 10 to 50 µl of oleic acid may be added relative to 1 ml of skim milk. For the whole milk, if a larger amount of oleic acid is added, the activity of the denatured product of lactalbumin of the present invention in inducing cell death in tumor cells can be enhanced. For the skim milk, an enhancement proportional to the amount of addition in the activity of inducing cell death in tumor cells was not recognized, even though oleic acid was added in excess.

The denatured products of lactalbumin which are produced by the above-described reaction, particularly the cell death inducing substance which induces cell death in tumor cells, may be directly used after the reaction, or may also be used after having sugar fractions or lipid fractions removed by known methods.

### Brief Description of the Drawings

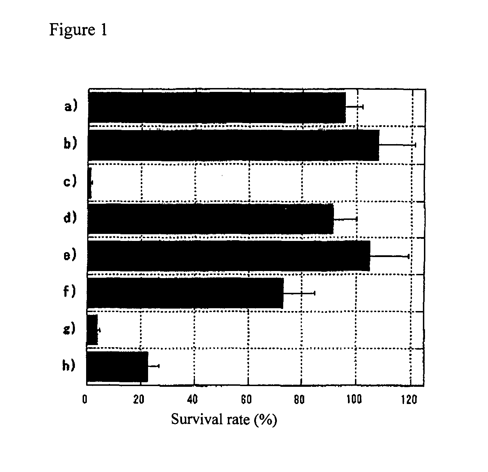

Fig. 1 shows the results of a comparison test for the cell death inducing activities of the cell death inducing substance produced by the method of the present invention and of Comparative Example. Reference numerals a) to h) in the figure correspond to the reference numerals a) to h) of the samples shown in Test Example 2), respectively.

Hereinafter, specific aspects of the present invention will be described in more detail by way of Examples, but the present invention is not intended to be limited by such Examples.

### Example 1

12 µl of oleic acid was added to 500 µl of commercially available milk and mixed, and then the mixture was heated for 10 minutes in an oil bath at 60°C. During the heating, the milk was stirred repeatedly at an interval of 2 minutes. The milk removed from the oil bath was left to stand at room temperature for 1 hour, and then was centrifuged for 5 minutes at 6200 rpm to recover an aqueous fraction in the lower layer. Thus, the denatured products of lactalbumin (cell death inducing substance) were obtained.

### Example 2

Skim milk was prepared by centrifuging commercially available milk for 10 minutes at 4700 rpm followed by recovering the supernatant fraction. 4 µl of oleic acid was added to 500 µl of the skim milk and mixed, and then the mixture was heated for 10 minutes in an oil bath at 60°C. During the heating, the skim milk was stirred repeatedly at an interval of 2 minutes. The skim milk removed from the oil bath was left to stand at room temperature for 1 hour, and then was centrifuged for 5 minutes at 6200 rpm to recover an aqueous fraction in the lower layer. Thus, the denatured products of lactalbumin (cell death inducing substance) were obtained.

### <Test Example>

### 1) Method for verifying cell death inducing activity

L1210 cells which are mouse-derived leukemic lymphocytes were suspended in RPMI 1640 medium (manufactured by Sigma-Aldrich Company) to prepare a cell suspension at a concentration of 2 × 10⁶ cells/ml. 90 µl of the cell suspension and 10 µl of a test sample were added to each well of a 96-well plate, and the plate was incubated at 37°C in a 5% CO₂ atmosphere. 30 minutes after the initiation of incubation, 10 µl of FBS (Fetal Bovine Serum) was added to each well, and incubation was further continued at 37°C in a 5% CO₂ atmosphere. Three hours after the addition of an aqueous layer, the L1210 cells were recovered, and Trypan blue staining was performed to investigate the survival rate of cells. The survival rate was calculated as the ratio of the number of live cells in each of the test wells, relative to the total number of cells in the wells added with phosphate buffered physiological saline instead of the aqueous layer.

### 2) Preparation of test samples

The following samples were provided as samples to be tested.

a) Control (phosphate buffered physiological saline)
b) Commercially available milk (untreated)
c) Tumor cell death inducing substance produced in Example 1
d) Skim milk prepared in Example 2
e) Sample prepared by the method of Example 2, except that heating at 60°C was replaced by standing still at room temperature
f) Sample produced by the method of Example 2, except that the heating temperature was set to 50°C and the heating time was set to 5 minutes
g) Cell death inhibiting substance produced in Example 2
h) Sample treated under the same conditions as in Example 2, except that the skim milk prepared in Example 2 was replaced by phosphate buffered physiological saline containing 1.5 mg of bovine lactalbumin

3) Verification of cell death inducing activity The results for the measurement of the cell death inducing activities of the respective samples provided in section 2) by the method shown in section 1), are presented in Fig. 1.

As clearly seen from these results, the presence of a cell death inducing substance was not recognized in the samples which were prepared only by adding oleic acid to milk or skim milk, without heating. On the other hand, the samples prepared by adding oleic acid to milk or skim milk and heating the mixture (Examples 1 and 2), exhibited significantly strong cell death inducing activity, even compared to the sample h) which was prepared by heating bovine lactalbumin and oleic acid together.

## Claims

1. A method for producing the denatured products of albumin, the method comprising heating a mixture of domestic animal milk and oleic acid.

2. The producing method according to claim 1, wherein the milk is skimmed milk.

3. The producing method according to claim 1, wherein the domestic animal is a cow.

4. The producing method according to claim 1, wherein the mixture is heated at 50°C to 60°C.

5. The producing method according to claim 1, further comprising centrifuging the heated mixture and recovering an aqueous layer.

6. The producing method according to any one of claims 1 to 5, wherein the denatured lactalbumin exhibiting cell death inducing activity against tumor cells is produced.
